# EUROPEAN PATENT SPECIFICATION

(11) **EP 3 720 541 B1**
(45) Date of publication and mention of the grant of the patent: **27.08.2025**
(21) Application number: 18839896.0
(22) Date of filing: 20.11.2018
(51) Int. Cl.: A61M 39/10

(54) **MULTI-LUMEN LUER CONNECTOR**
MULTI-LUMEN-LUER-VERBINDER
RACCORD LUER À LUMIÈRES MULTIPLES

(30) Priority: 04.12.2017 NL 2020013
(43) Date of publication of application: 14.10.2020
(73) Proprietor: Crea IP B.V., 3237 MG Vierpolders (NL)
(72) Inventor: DAM-HUISMAN, Adriaantje Coliene, 2645 BA Delfgauw (NL)
(74) Representative: Nederlandsch Octrooibureau
(86) International application number: PCT/NL2018/050778
(87) International publication number: WO 2019/112420

(56) References cited:
- EP-A1- 2 783 724
- EP-A2- 1 992 861
- WO-A1-2009/081178
- US-A1- 2014 296 835

## Description

### Field of the invention

The present invention relates to a multi-lumen luer connector, and more particularly to a coaxial multi-lumen connector for use in ophthalmic surgical systems and apparatus.

### Background art

Systems for use in ophthalmic surgery often comprise machines that provide for delivery or exchange of multiple fluids to a surgical site. For example, the systems may allow controlled aspiration of fluid from a surgical site (e.g. the eye) and irrigation of the surgical site to replace the aspirated fluid. In order to deliver fluid to and from the eye, fluid conduits extend from a machine that controls the flow of fluid to an aspiration/irrigation tip. The fluid conduits can be connected to the machine using luer-type connections. Luer-type connections typically have a tapered nozzle (male part) with a bore extending therethrough and a conical bore (female part) configured to receive the nozzle and form a seal therewith. Luer-type connections have been used in several medical applications.

US Patent Application Publication No. US2007/0076401 describes a coupling connection used in enteral nutrition lines, which uses luer-lock type connections securable with a screw connector to prevent axial decoupling of the luer parts. The connector joins two lumens to provide a single fluid conduit.

PCT Application No. WO2009/081178 describes a connector for connecting two conduits, each having a plurality of lumens passing therethrough. The connector comprises luer-type connecting parts that couple the lumens side by side. The connector includes retaining means for holding the two connecting parts together, which comprise latching arms one of the connecting parts adapted to engage a recess on the other connecting part when the connecting parts are pushed together.

### Summary of the invention

The present invention seeks to provide a luer connector for connecting multiple fluid conduits with a single connector.

According to the present invention, there is provided a multi-lumen luer connector comprising: a first part having a first outer connector and a first inner connector disposed within the first outer connector, and a second part having a second outer connector and a second inner connector disposed within the second outer connector, wherein one of the first and second outer connectors is configured as a male part comprising a first tapered body having a bore extending therethrough and the other of the first and second outer connectors is configured as a female part comprising a first conical bore adapted to receive the first tapered body and sealingly engage therewith to define an outer lumen, and wherein one of the first and second inner connectors is configured as a male part comprising a second tapered body having a bore extending therethrough and the other of the first and second inner connectors is configured as a female part comprising a second conical bore configured to receive the second tapered body and sealingly engage therewith to define an inner lumen disposed within the outer lumen.

Further embodiments are described in the dependent claims.

The multi-lumen connector described herein advantageously allows two fluid lines to be connected with a single connector. By providing a single connector for coupling multiple conduits, the time required to connect multiple fluid lines is reduced. Moreover, the multi-lumen configuration of the present invention eliminates the risk of fluid lines being connected incorrectly due to crossed tubing. Advantageously, a coaxial arrangement of luer connectors allows connection and disconnection of the first and second parts with a twisting motion, which is familiar to healthcare practitioners in the field. Other advantages associated with the present invention will be apparent to the skilled person from the following detailed description.

### Short description of drawings

Exemplary embodiments of the invention will now be described with reference to the attached drawings, in which:
Fig. 1A shows a longitudinal cross-section of a multi-lumen luer connector according to an embodiment of the present invention;
Fig. 1B shows a longitudinal cross-section a multi-lumen luer connector according to another embodiment the present invention;
Fig. 2 shows a transverse cross-section of the multi-lumen luer connector shown in Figs. 1A and B;
Fig. 3 is a perspective view of the multi-lumen luer connector shown in Fig. 1A; and
Fig. 4 shows an ophthalmic surgical apparatus comprising the connector of Figs. 1A and 1B.

### Description of embodiments

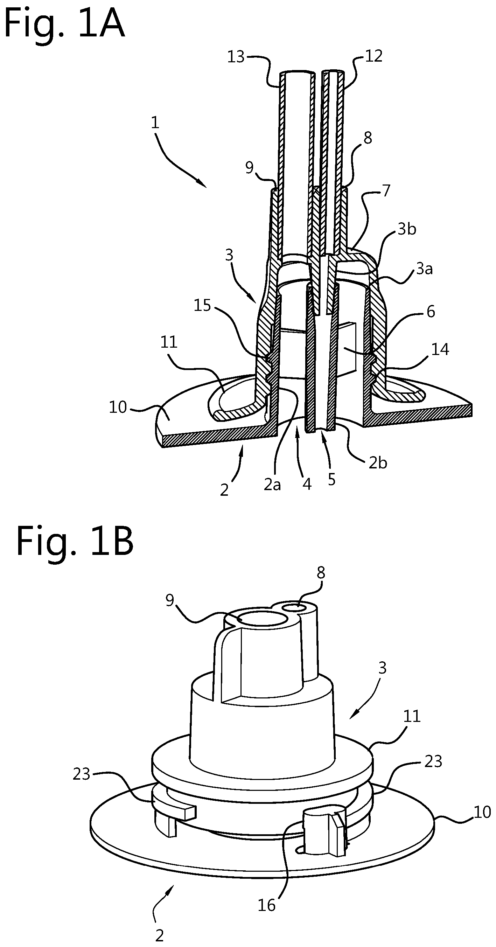

Figure 1 shows a multi-lumen luer connector in accordance with an exemplary embodiment of the present invention. As shown in figure 1, the multi-lumen luer connector 1 comprises a first part 2 having a first outer connector 2a and a first inner connector 2b disposed within the first outer connector 2a. The multi lumen connector 1 further comprises a second part 3 having a second outer connector 3a and a second inner connector 3b disposed within the second outer connector 3a. One of the first and second outer connectors 2a, 3a is configured as a male part comprising a first tapered body having a bore extending therethrough and the other of the first and second outer connectors 2a, 3a is configured as a female part comprising a first conical bore adapted to receive the first tapered body (e.g. a first tapered nozzle) and sealingly engage therewith to define an outer lumen 4. Similarly, one of the first and second inner connectors 2b, 3b is configured as a male part comprising a second tapered body (e.g. a second tapered nozzle) having a bore extending therethrough and the other of the first and second inner connectors 2b, 3b is configured as a female part comprising a second conical bore configured to receive the second tapered body and sealingly engage therewith to define an inner lumen 5 disposed within the outer lumen 4. In other words, the first and second outer connectors 2a, 3a are configured as a first male-female pair that sealingly engage with each other to form the outer lumen 4 and the first and second inner connectors 2b, 3b are configured as a second male-female pair that sealingly engage with each other to form the inner lumen 5, which is disposed within the outer lumen 4.

The male and female connection parts of the first and second parts 2, 3 can comprise any material known in the art that as suitable for standard single-lumen luer connectors. For example, the first and second parts can comprise acrylonitrile butadiene styrene (ABS), polyvinyl chloride (PVC), polyethylene or other common plastics. Advantageously, the materials chosen for the first and second parts are at least slightly deformable to facilitate a fluid tight seal between the male and female parts of the connector 1. In at least some embodiments, the first part 2 is integrally formed as part of a surgical cassette, e.g. an irrigation and/or aspiration cassette used in ophthalmic surgery. In these embodiments, the first part 2 can be molded as a single piece with the cassette body (not shown). In other embodiments, the first part 2 can be provided as a separate component that can be mated with a port on a surgical cassette. In some embodiments, the invention comprises the second part 3 as an individual component, without the first part 2. In other embodiments, the invention comprises the first part 2 as an individual component, without the second part 3.

The male-female pairs in the multi-lumen luer connector 1 can be configured in different ways. In some embodiments of the invention, the first outer connector 2a and the first inner connector 2b can be configured as male parts and the second outer connector 3a and the second inner connector 3b can be configured as complementary female parts.

In other embodiments, each of the first and second parts 2, 3 can be configured with one male part and one female part. For example, as shown in Figure 1, the first outer connector 2a and the second inner connector 3b can be configured as male parts and the second outer connector 3a and the first inner connector 2b can be configured as female parts. The skilled person will appreciate that the inverse is also possible (the first inner connector 2b and the second outer connector 3a can be configured as male connectors and the first outer connector and the second inner connector can be configured as female connectors).

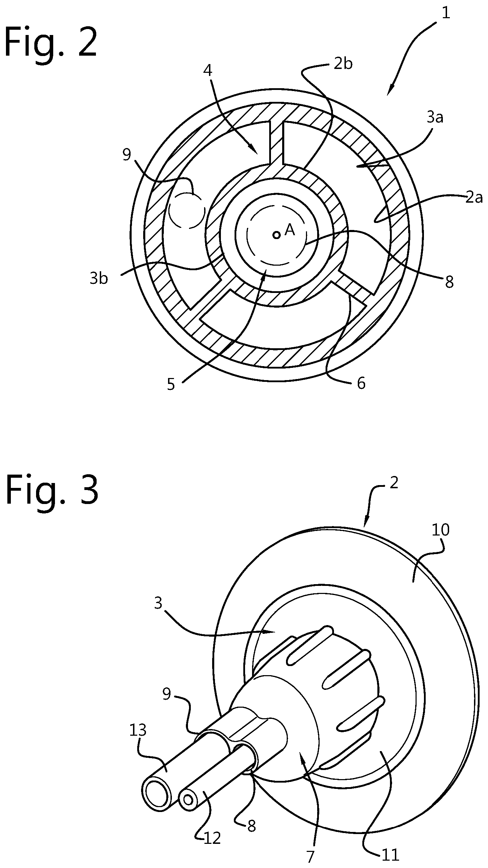

As shown in Figure 1, the inner lumen 5 can be disposed coaxially within the outer lumen 4. In the exemplary embodiment shown in Figure 1, the inner lumen 5 shares a central longitudinal axis A (see also Figure 2) with the outer lumen 4. However, the skilled person will appreciate that the inner lumen 5 may be arranged within the outer lumen 4 such that its longitudinal axis offset from the central axis A of the outer lumen 4.

The coaxial arrangement shown in Figure 1 is particularly advantageous because it allows the first and second parts 2, 3 to be connected and disconnected from each other using a twisting motion (in the same manner as a standard single lumen luer), which is familiar to healthcare professionals. This is particularly advantageous in the case of luer-lock embodiments, in which male and female luer connectors are locked together, conventionally by twisting the male part relative to the female part. Conventionally, a partial turn (e.g. a quarter turn or half turn) of the male part relative to the female part is used to lock the parts together, by means of threaded engagement, bayonet engagement or other locking means that will be apparent to the person skilled in the art. Of course the skilled person will appreciate that a full turn or multiple turns can be used to engage the male and female parts with each other. Even in slip-fit embodiments (non-locking embodiments) the first part 2 is commonly twisted relative to the second part 3 during connection and disconnection, to facilitate fluid tight engagement between the first and second parts.

In some embodiments of the invention, at least one of the male/female pairs of the multi-lumen luer connector 1 can be configured as a slip fit luer connection. In a slip-fit luer connection, a frictional seal is formed between the male and female components, which is substantially fluid-tight. To connect and disconnect the first and second parts from each other, a user simply pushes the tapered body of the male part into the conical bore of the female part. In a slip fit luer connection, the tapered body and/or the conical bore can be configured with substantially smooth sides. Alternatively, surface features may be provided on the outer surface of the tapered body or the inner surface of the conical bore to provide fluid tight frictional engagement between the male and female parts. For example, the tapered body may be provided with circumferential ridges or ribs configured to engage complementary grooves on the female part. The ridges may be formed of a resiliently deformable material, such as rubber or silicone, which can be pushed into the grooves on the female part.

In other embodiments, at least one of the male-female pairs can be configured as a luer-lock type connector. In a luer-lock connector according to the present invention, the male and/or female parts is provided with additional means for locking the male and female parts in a sealing engagement. For example, at least one of the male/female pairs can be further configured for threaded engagement.

As shown in Figure 1A, the first outer connector 2a can comprise at least one thread 14. The second outer connector 3a comprises a complementary groove 15 into which the thread 14 can be screwed. The engagement of the thread 14 and the groove 15 securely locks the first and second outer connectors 2a, 3a together. Similarly, the first and second inner connectors 2b, 3b can also be provided with a threaded engagement. In this configuration, the first and second parts 2, 3 can be secured together by way of a quarter turn of the first part 2 relative to the second part 3. This step is familiar to healthcare professionals as the manner in which single lumen luer connectors are coupled. The skilled person will appreciate that in embodiments of the present invention one or both of the male-female pairs can be adapted with thread engagement.

In an alternative embodiment shown in Figure 1B, the first part 2 can comprise one or more claws 16 configured to engage a thread(s) 23 on the outer surface of the second part 3. The thread(s) 23 can be helical (as in a conventional screw) such that rotation of the first part 2 relative to the second part 3 causes relative axial movement of the first and second parts 2, 3. The embodiment shown in Figure 1B can be advantageous because the locking engagement between the first and second parts 2, 3 is visible to the user and incorrect connections can be identified by the user by sight. Moreover, the manufacturing process for the claw-thread engagement shown in Figure 1B is favourable.

In an alternative luer-lock type engagement, at least one of the male-female pairs can be further configured for bayonet engagement. [claim 7]. As an example, in at least one embodiment, the first outer connector 2a may comprise a pair of diametrically opposed lugs that extend radially outwardly from its outer surface. The second outer connector 3a can comprise a pair of diametrically opposed L-shaped grooves or recesses configured to receive and engage the diametrically opposed lugs to lock the first and second outer connectors 2a, 3a together. In a similar manner to the threaded engagement described above, the first and second parts 2, 3 can be secured together by a quarter turn of the first part 2 relative to the second part 3. The skilled person will appreciate that other luer-lock type engagement may be possible.

The skilled person will understand that the present invention encompasses embodiments in which both the inner and outer connections are configured as slip-fit type luer connections, embodiments in which both connections are luer lock-type connections (e.g. with a threaded or bayonet fitting) or with one lock fit and one slip fit. In the latter case, it may be more convenient to provide the larger, outer connection with a luer-lock type engagement and the smaller, inner connection with a slip-fit engagement.

Figure 2 shows a cross-sectional view of the multi-lumen connector 1. As shown in Figure 2, the first part 2 can comprise at least one support structure 6 extending between the first outer connector 2a and the first inner connector 2b to support the inner connector 2b in the coaxial position. In the exemplary embodiment shown in Figure 2, three support structures 6 are provided to hold the first inner connector 2b centrally within the outer lumen 4 defined by the outer connector 2a. The support structures can take any form but must not prevent the passage of fluid through the outer lumen 4 defined by the outer connector 2a.

As shown in Figure 1 and also in the perspective view of Figure 3, the second part 3 can comprise an end wall 7 at its distal end, which comprises a first opening 8 in fluid communication with the inner lumen 5 and a second opening 9 in fluid communication with the outer lumen 4. [claim 9] The first and second openings 8, 9 can be configured for connection to first and second fluid delivery lines 12, 13 respectively. The skilled person will appreciate that one or both of the openings 8, 9 can be provided in a side wall of the second part 3.

As shown in Figure 1, the first opening 8 can be arranged coaxially with the first and second lumens 4, 5 and the second opening 9 can be arranged adjacent to the first opening 8 offset from the shared axis A of the inner and outer lumens 4,5. As shown in the perspective view of Figure 3, this configuration can advantageously allow the connector 1 to act as an adapter for connecting coaxial lumens 4, 5 to separate adjacent fluid lines 12, 13. The arrangement shown in Figure 3 is particularly advantageous because it eliminates the risk of two adjacent fluid conduits being connected to the wrong ports due to crossed lines or other human error. This prevents an irrigation tip being accidentally connected to an aspiration source part way through a procedure as a result of incorrect connection of fluid lines.

As shown in Figure 1, the first and second openings 8, 9 can be different sizes. For example, in the embodiment shown in Figures 1 to 3, the second opening 9 is larger than the first opening 8. The skilled person will understand that the size of the first and second openings 8 and 9 can be optimised with respect to the flow area of the first and second conduits and the desired flow characteristics across the connector 1. The relative flow areas of the first and second conduits can also be optimised depending on the desired application. For example, the second opening and the outer conduit can be configured as an irrigation conduit with a larger flow area than the inner conduit and first opening, which can be configured for aspiration.

Referring again to Figure 3, at least one of the first part 2 and the second part 3 comprises a flange 10, 11 extending outwardly from its outer surface. A flange 11 can be provided on the second part 3. A flange 11 provided on the second part 3 is advantageous because it prevents the user's finger from sliding forward along the connector to contact the second part 2. During use, it is advantageous to avoid contact with the first part 2 to maintain sterility of the surgical cassette and the fluid moving therethrough, to and from the eye. As an additional feature, or as an alternative, the second part 3 can also be provided with one or more grip enhancing features (not shown) on its outer surface to improve the user's grip to help prevent the user's fingers slipping towards the sterile surgical cassette. For example, the grip enhancing features can comprise an embossed or ridged region on the outer surface of the second part 3 that serve to increase the frictional engagement between the user's fingers and the second part 3. The grip enhancing features can also comprise a silicone sheath surrounding at least a part of outer surface of the second part 3. Other grip enhancing features will be apparent to the skilled person.

A flange can also be provided on the first part 2. In embodiments in which the first part 2 is formed as a separate component configured for mating with a surgical cassette, the flange can be provided as a radially extending surface 10 from the outer edge of the first part 2. The surface 10 can be provided flush with an outer surface of the surgical cassette or it can be set forward from a front surface of the surgical cassette. In embodiments in which the first part 2 is formed integrally with a surgical cassette, surface 10 can be a front surface of the surgical cassette.

The flange 11 advantageously provides a shield to prevent the user's fingers coming into contact with the surgical cassette during connection and disconnection of the luer, thereby maintaining the sterility of the surgical cassette. Should a fluid line need to be disconnected and/or reconnected during a surgical procedure, a user can disconnect and/or reconnect the first and second parts 2, 3 without touching the first and second outer connectors 2a, 3a (and the first and second inner connectors 2b, 3b) or the cassette. In at least one exemplary embodiment, as shown in Figure 3, the first part 2 comprises a surface 10 and the second part 3 comprises a flange 11. In this embodiment, the first and second parts 2, 3 are configured such that when the first and second parts 2, 3 are connected, the surface 10 and an opposing face of the flange 11 are spaced apart from each other in an axial direction (i.e. the flange 11 does not abut the surface 10). This can be implemented by purposely selecting the dimensions of the relevant components of the first and second part 2, 3. e.g., the intended overlap of the first and second outer connectors 2a, 3a, and the first and second inner connectors 2b, 3b, respectively, a longitudinal length of the threaded/bayonet engagement of first and second parts 2, 3 and the positioning of the flange 11 may be selected to ensure that the flange 11 and the surface 10 are spaced from each other when the first and second male and female parts 2a,b, 3a,b are fully engaged. By engineering the connector such that a space is left between the flange 11 and the surface 10 when the connector 2,3 is fully engaged, abutment of the flange 11 and the surface 10 will not limit the degree of engagement of the first and second parts 2, 3.

Although the multi-lumen connector 1 described above with reference to Figures 1 to 3 comprises two coaxial lumens, the skilled person will appreciate that a multi-lumen connector according to the present invention may be provided with three or more coaxial lumens. For example, the first part 2 may comprise a further connector (not shown) disposed within the first inner connector 2b. The second part 3 may also comprise a further connector disposed within the second inner connector 3b. These further connectors can be configured as a male-female luer pair as described above to provide a third lumen coaxially disposed within the inner lumen 4.

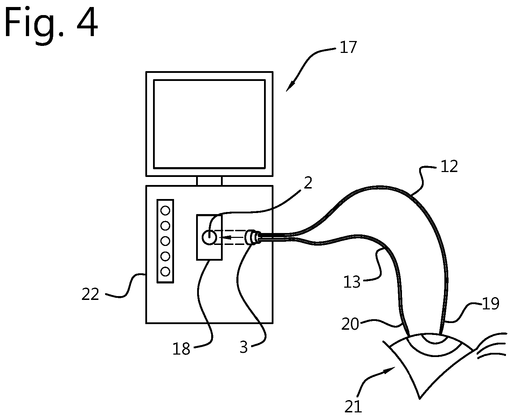

It is envisaged that the multi-lumen luer connector 1 described herein can be used for surgical aspiration and/or irrigation to an eye, as shown in Figure 4. In at least some embodiments of the invention, the multi lumen luer connector 1 is provided with a first fluid line 12 fluidically connected to the outer lumen 4 and a second fluid line 13 fluidically connected to the inner lumen 5. The first and second fluid lines 12, 13 can be provided at their distal ends with surgical tips, e.g. a irrigation tip and/or an aspiration tip.

The multi-lumen connector 1 and the fluid lines 12, 13 can be comprised in a surgical irrigation and/or aspiration apparatus. As shown in Figure 4, an ophthalmic surgical system 17 can comprise the multi-lumen connector 1 described above with the first part 2 provided in a housing of a surgical cassette 18. The surgical cassette can comprise a first port and a second port.

As shown in Figure 4, the second part 3 is coupled to a first fluid line 12 via the first opening 8 and a second fluid line 13 via the second opening 9. The first and second fluid lines 12, 13 are in turn coupled to a first surgical tip 19 and a second surgical tip 20 respectively. The outer and inner lumens 4, 5 of the first part 2 are fluidically connected to the first and second ports of the surgical cassette 18. The first and second ports of the surgical cassette can fluidically connect the surgical tips with respective fluid sources/drains. For example, the the first port and the second port can open into first and second chambers of the surgical cassette 18 respectively, each configured to a hold a different irrigation fluid (e.g. a first irrigation liquid and a second irrigation liquid, or a first irrigation liquid and a source of air for irrigation). Alternatively, the first and second ports can be configured to open into a single chamber of a surgical cassette 18. In such embodiments, the first port can open into a lower part of the chamber (filled with irrigation liquid), whilst the second port can open into an upper part of the chamber (filled with air). In some embodiments, the surgical cassette may not comprise a chamber and can instead comprise a network of channels that fluidically couple the first and second ports to external fluid sources (e.g. an infusion bottle). A similar arrangement can be used for combined irrigation and aspiration applications (e.g. wherein the first port is in fluid communication with an infusion source and a positive pressure for delivering fluid to the eye, whilst the second port is in fluid communication with a drain and a negative pressure source for aspirating fluid from the eye).

In the system shown in Figure 4, the first part 2 provides a convenient external connector on the housing of the cartridge 18 which can be inserted into a machine 22 for controlling the flow of fluid to and from the eye. The second part 3, which is coupled to the first and second conduits 12, 13 can be easily plugged into the first part 2 to connect the first and second surgical tips 19, 20 (e.g. an irrigation tip and an aspiration tip) to the appropriate port/chamber. During use, should the first and second lines 12, 13 become blocked, or should the type of tip assembly require changing, the surgeon can simply disconnect the second part 3 from the first part 2 and connect a new second part 3, which is connected via new fluid lines 12, 13 to the appropriate surgical tips 19, 20.

As can be seen in Figure 1, the connector 1 connects the coaxial lumens 4, 5 into separate fluid lines 12, 13 for delivery fluid to or from the surgical site (e.g. the eye as shown in Figure 4). However, the skilled person will recognise that the openings 8 and 9 in the end wall 7 of the second part 3 can be configured to connect to a coaxial fluid conduit (not shown).

In the exemplary embodiment described with reference to Figure 4, the first and second fluid lines 12, 13 are configured for use in an ophthalmic surgical procedure and have an inner diameter of between 0.5mm and 9mm, e.g. 1.5mm, e.g. 4mm. However, the skilled person will appreciate that the advantages associated with the present invention are not limited to ophthalmic surgical systems. To the contrary, the present invention may be usefully employed in other medical applications (e.g. connections for enteral feeding tubes) and non-medical applications (e.g. fine plant irrigation systems) in which multiple fluid lines must be easily and conveniently connected.

The present invention has been described above with reference to a number of exemplary embodiments as shown in the drawings. The skilled person will appreciate that modifications and alternative implementations of some parts or elements are possible, and are included in the scope of protection as defined in the appended claims.

## Claims

1. A multi-lumen luer connector (1) comprising:
a first part (2) having a first outer connector (2a) and a first inner connector (2b) disposed within the first outer connector (2a);
a second part (3) having a second outer connector (3a)
wherein one of the first and second outer connectors (2a, 3a) is configured as a male part comprising a first tapered body having a bore extending therethrough and the other of the first and second outer connectors (2a, 3a) is configured as a female part comprising a first conical bore adapted to receive the first tapered body and sealingly engage therewith to define an outer lumen (4),
**characterized in that** a second inner connector (3b) is disposed within the second outer connector (3a); and **in that** one of the first and second inner connectors (2b, 3b) is configured as a male part comprising a second tapered body having a bore extending therethrough and the other of the first and second inner connectors (2b, 3b) is configured as a female part comprising a second conical bore configured to receive the second tapered body and sealingly engage therewith to define an inner lumen (5) disposed within the outer lumen (4).

2. The multi-lumen luer connector (1) according to claim 1, wherein the inner lumen (5) is disposed coaxially within the outer lumen (4).

3. The multi-lumen luer connector (1) according to claim 1 or claim 2, wherein the first outer connector (2a) and the first inner connector (2b) are configured as male parts and the second outer connector (3a) and the second inner connector (3b) are configured as female parts.

4. The multi-lumen luer connector (1) according to claim 1 or claim 2, wherein the first outer connector (2a) and the second inner connector (3b) are configured as male parts and the second outer connector (3a) and the first inner connector (2b) are configured as female parts.

5. The multi-lumen luer connector (1) according to any one of claims 1 to 4, wherein at least one of the male-female pairs is configured as a slip fit luer connection.

6. The multi-lumen luer connector (1) according to any one of claims 1 to 5, wherein at least one of the male-female pairs is further configured for threaded engagement.

7. The multi-lumen luer connector (1) according to any one of claims 1 to 6, wherein at least one of the male-female pairs is further configured for bayonet engagement.

8. The multi-lumen luer connector (1) according to any of claims 1 to 7, wherein the first part (2) comprises at least one support structure (6) extending between the first outer connector (2a) and the first inner connector (2b) to support the inner connector (2b) in the coaxial position.

9. The multi-lumen luer connector (1) according to any one of claims 1 to 8, wherein the second part (3) comprises an end wall (7) at its distal end, the end wall (7) comprising a first opening (8) in fluid communication with the inner lumen (5) and a second opening (9) in fluid communication with the outer lumen (4).

10. The multi-lumen luer connector (1) according to claim 9, wherein the first opening (8) is arranged coaxially with the first and second lumens (4, 5) and the second opening (9) is arranged adjacent to the first opening (8) offset from the shared axis of the inner and outer lumens (4,5).

11. The multi-lumen luer connector (1) according to any one of claims 1 to 10, wherein at least one of the first part (2) and the second part (3) comprises a flange (11) extending outwardly from its outer surface.

12. The multi-lumen connector (1) according to any one of claims 1 to 11, wherein the first connector (2) comprises a surface (10) and the second connector (3) comprises a flange (11), wherein the first and second parts (2,3) are configured such that when the first and second parts (2, 3) are connected, the surface (10) and an opposing face of the flange (11) are spaced apart from each other in an axial direction.

13. The multi-lumen connector (1) according to any one of claims 1 to 12, wherein:
the first part (2) comprises a first further connector disposed within the first inner connector (2b);
the second part (3) comprises a second further connector disposed within the second inner connector (3b) to provide a third lumen coaxially disposed within the inner lumen (4).

14. The multi-lumen luer connector (1) according to any one of the preceding claims, further comprising a first fluid line (12) fluidically connected to the outer lumen (4) and a second fluid line (13) fluidically connected to the inner lumen (5).

15. A system (17) for ophthalmic surgery comprising:
the multi-lumen connector (1) according to any one of claims 1 to 14, wherein:
the first part (2) is provided in a housing of a surgical cassette (18) comprising a first port and a second port; and
the second part is coupled to a first fluid line (12) via the first opening (8) and a second fluid line (13) via the second opening (9); and
wherein the first and second fluid lines (12, 13) are coupled to a first surgical tip (19) and a second surgical tip (20) respectively, and wherein the outer and inner lumens (4, 5) of the first part (2) are fluidically connected to the first and second ports of the surgical cassette (18).

## Patentansprüche

1. Mehrlumiger Luer-Verbinder (1) umfassend:
ein erstes Teil (2) mit einem ersten äußeren Verbinder (2a) und einem ersten inneren Verbinder (2b), der innerhalb des ersten äußeren Verbinders (2a) angeordnet ist;
ein zweites Teil (3) mit einem zweiten äußeren Verbinder (3a),
wobei einer der ersten und zweiten äußeren Verbinder (2a, 3a) als ein männliches Teil ausgebildet ist, das einen ersten konischen Körper mit einer sich durch diesen hindurch erstreckenden Bohrung aufweist, und der andere der ersten und zweiten äußeren Verbinder (2a, 3a) als ein weibliches Teil ausgebildet ist, das eine erste konische Bohrung umfasst, die angepasst ist, um den ersten konischen Körper aufzunehmen und abdichtend mit diesem in Eingriff zu treten, um ein äußeres Lumen (4) zu definieren,
**dadurch gekennzeichnet, dass** ein zweiter innerer Verbinder (3b) innerhalb des zweiten äußeren Verbinders (3a) angeordnet ist; und dass einer der ersten und zweiten inneren Verbinder (2b, 3b) als ein männlicher Teil ausgebildet ist, der einen zweiten konischen Körper mit einer sich durch diesen hindurch erstreckenden Bohrung aufweist, und der andere der ersten und zweiten inneren Verbinder (2b, 3b) als ein weiblicher Teil ausgebildet ist, der eine zweite konische Bohrung umfasst, die dazu eingerichtet ist, den zweiten konischen Körper aufzunehmen und abdichtend mit diesem in Eingriff zu kommen, um ein inneres Lumen (5) zu definieren, das innerhalb des äußeren Lumens (4) angeordnet ist.

2. Mehrlumiger Luer-Verbinder (1) nach Anspruch 1, wobei das innere Lumen (5) koaxial innerhalb des äußeren Lumens (4) angeordnet ist.

3. Mehrlumiger Luer-Verbinder (1) nach Anspruch 1 oder Anspruch 2, wobei der erste äußere Verbinder (2a) und der erste innere Verbinder (2b) als männliche Teile ausgebildet sind und der zweite äußere Verbinder (3a) und der zweite innere Verbinder (3b) als weibliche Teile ausgebildet sind.

4. Mehrlumiger Luer-Verbinder (1) nach Anspruch 1 oder Anspruch 2, wobei der erste äußere Verbinder (2a) und der zweite innere Verbinder (3b) als männliche Teile ausgebildet sind und der zweite äußere Verbinder (3a) und der erste innere Verbinder (2b) als weibliche Teile ausgebildet sind.

5. Mehrlumiger Luer-Verbinder (1) nach einem der Ansprüche 1 bis 4, wobei mindestens eines der männlich-weiblichen Paare als Spielpassungs-Luer-Verbinder ausgebildet ist.

6. Mehrlumiger Luer-Verbinder(1) nach einem der Ansprüche 1 bis 5, wobei mindestens eines der männlich-weiblichen Paare außerdem für einen Gewindeeingriff ausgebildet ist.

7. Mehrlumiger Luer-Verbinder (1) nach einem der Ansprüche 1 bis 6, wobei mindestens eines der männlich-weiblichen Paare außerdem für einen Bajonettverschluss ausgebildet ist.

8. Mehrlumiger Luer-Verbinder (1) nach einem der Ansprüche 1 bis 7, wobei das erste Teil (2) mindestens eine Stützstruktur (6) umfasst, die sich zwischen dem ersten äußeren Verbinder (2a) und dem ersten inneren Verbinder (2b) erstreckt, um den inneren Verbinder (2b) in der koaxialen Position zu stützen.

9. Mehrlumiger Luer-Verbinder (1) nach einem der Ansprüche 1 bis 8, wobei das zweite Teil (3) an seinem distalen Ende eine Endwand (7) umfasst, wobei die Endwand (7) eine erste Öffnung (8) in Fluidverbindung mit dem inneren Lumen (5) und eine zweite Öffnung (9) in Fluidverbindung mit dem äußeren Lumen (4) umfasst.

10. Mehrlumiger Luer-Verbinder (1) nach Anspruch 9, wobei die erste Öffnung (8) koaxial mit dem ersten und zweiten Lumen (4, 5) angeordnet ist und die zweite Öffnung (9) benachbart zu der ersten Öffnung (8) versetzt zur gemeinsamen Achse des inneren und äußeren Lumens (4, 5) angeordnet ist.

11. Mehrlumiger-Luer-Verbinder (1) nach einem der Ansprüche 1 bis 10, wobei mindestens eines der beiden Teile, des ersten Teils (2) und des zweiten Teils (3) einen Flansch (11) umfasst, der sich von seiner Außenfläche nach außen erstreckt.

12. Mehrlumiger Verbinder (1) nach einem der Ansprüche 1 bis 11, wobei der erste Verbinder (2) eine Oberfläche (10) umfasst und der zweite Verbinder (3) einen Flansch (11) umfasst, wobei das erste und das zweite Teil (2, 3) so eingerichtet sind, dass, wenn das erste und das zweite Teil (2, 3) verbunden sind, die Oberfläche (10) und eine gegenüberliegende Fläche des Flansches (11) in einer axialen Richtung voneinander beabstandet sind.

13. Mehrlumiger Verbinder (1) nach einem der Ansprüche 1 bis 12, wobei:
das erste Teil (2) einen ersten weiteren Verbinder umfasst, der innerhalb des ersten inneren Verbinders (2b) angeordnet ist;
das zweite Teil (3) einen zweiten weiteren Verbinder umfasst, der innerhalb des zweiten inneren Verbinders (3b) angeordnet ist, um ein drittes Lumen zu bilden, das koaxial innerhalb des inneren Lumens (4) angeordnet ist.

14. Mehrlumiger Luer-Verbinder (1) nach einem der vorstehenden Ansprüche ferner umfassend eine erste Fluidleitung (12), die mit dem äußeren Lumen (4) fluidisch verbunden ist, und eine zweite Fluidleitung (13), die mit dem inneren Lumen (5) fluidisch verbunden ist.

15. System (17) für die Augenchirurgie umfassend:
den mehrlumigen Verbinder (1) nach einem der Ansprüche 1 bis 14, wobei:
das erste Teil (2) in einem Gehäuse einer chirurgischen Kassette (18) vorgesehen ist, die einen ersten Anschluss und einen zweiten Anschluss umfasst; und
das zweite Teil über die erste Öffnung (8) mit einer ersten Fluidleitung (12) und über die zweite Öffnung (9) mit einer zweiten Fluidleitung (13) verbunden ist; und
wobei die erste und die zweite Fluidleitung (12, 13) mit einer ersten chirurgischen Spitze (19) bzw. einer zweiten chirurgischen Spitze (20) verbunden sind, und wobei das äußere und das innere Lumen (4, 5) des ersten Teils (2) mit dem ersten und dem zweiten Anschluss der chirurgischen Kassette (18) fluidisch verbunden sind.

## Revendications

1. Connecteur Luer à lumières multiples (1) comprenant :
une première partie (2) ayant un premier connecteur extérieur (2a) et un premier connecteur intérieur (2b) disposé à l'intérieur du premier connecteur extérieur (2a) ;
une deuxième partie (3) ayant un deuxième connecteur extérieur (3a)
où l'un du premier et deuxième connecteur extérieur (2a, 3a) est configuré comme une partie mâle comprenant un premier corps conique traversé par un alésage, et l'autre du premier et deuxième connecteur extérieur (2a, 3a) est configuré comme une partie femelle comprenant un premier alésage conique adapté pour recevoir le premier corps conique et s'y engager de manière étanche avec celui-ci afin de définir une lumière extérieure (4).
**caractérisé en ce qu'**un deuxième connecteur intérieur (3b) est disposé à l'intérieur du deuxième connecteur extérieur (3a) ; et **en ce que** l'un du premier et deuxième connecteur intérieur (2b, 3b) est configuré comme une partie mâle comprenant un deuxième corps conique traversé par un alésage, et l'autre du premier et deuxième connecteur intérieur (2b, 3b) est configuré comme une partie femelle comprenant un deuxième alésage conique configuré pour recevoir le deuxième corps conique et s'y engager de manière étanche avec celui-ci afin de définir une lumière intérieure (5) disposée à l'intérieur de la lumière extérieure (4).

2. Connecteur Luer à lumières multiples (1) selon la revendication 1, où la lumière intérieure (5) est disposée coaxialement à l'intérieur de la lumière extérieure (4).

3. Connecteur Luer à lumières multiples (1) selon la revendication 1 ou la revendication 2, où le premier connecteur extérieur (2a) et le premier connecteur intérieur (2b) sont configurés comme des parties mâles et le deuxième connecteur extérieur (3a) et le deuxième connecteur intérieur (3b) sont configurés comme des parties femelles.

4. Connecteur Luer à lumières multiples (1) selon la revendication 1 ou la revendication 2, où le premier connecteur extérieur (2a) et le deuxième connecteur intérieur (3b) sont configurés comme des parties mâles et le deuxième connecteur extérieur (3a) et le premier connecteur intérieur (2b) sont configurés comme des parties femelles.

5. Connecteur Luer à lumières multiples (1) selon l'une quelconque des revendications 1 à 4, où au moins l'une des paires mâle-femelle est configurée comme une connexion Luer à ajustement glissant.

6. Connecteur Luer à lumières multiples (1) selon l'une quelconque des revendications 1 à 5, où au moins l'une des paires mâle-femelle est en outre configurée pour un engagement fileté.

7. Connecteur Luer à lumières multiples (1) selon l'une quelconque des revendications 1 à 6, où au moins l'une des paires mâle-femelle est en outre configurée pour un engagement à baïonnette.

8. Connecteur Luer à lumières multiples (1) selon l'une quelconque des revendications 1 à 7, où la première partie (2) comprend au moins une structure de support (6) s'étendant entre le premier connecteur extérieur (2a) et le premier connecteur intérieur (2b) pour soutenir le connecteur intérieur (2b) dans la position coaxiale.

9. Connecteur Luer à lumières multiples (1) selon l'une quelconque des revendications 1 à 8, où la deuxième partie (3) comprend une paroi d'extrémité (7) à son extrémité distale, la paroi d'extrémité (7) comprenant une première ouverture (8) en communication fluidique avec la lumière intérieure (5) et une deuxième ouverture (9) en communication fluidique avec la lumière extérieure (4).

10. Connecteur Luer à lumières multiples (1) selon la revendication 9, où la première ouverture (8) est disposée coaxialement à la première et deuxième lumière (4, 5) et la deuxième ouverture (9) est disposée adjacente à la première ouverture (8), décalée par rapport à l'axe commun des lumières intérieure et extérieure (4, 5).

11. Connecteur Luer à lumières multiples (1) selon l'une quelconque des revendications 1 à 10, où au moins l'une parmi la première partie (2) et la deuxième partie (3) comprend une bride (11) s'étendant vers l'extérieur depuis sa surface extérieure.

12. Connecteur à lumières multiples (1) selon l'une quelconque des revendications 1 à 11, où le premier connecteur (2) comprend une surface (10) et le deuxième connecteur (3) comprend une bride (11), où la première et deuxièmes partie (2, 3) sont configurées de telle sorte que, lorsque la première et deuxième partie sont connectées, la surface (10) et une face opposée de la bride (11) sont espacées l'une de l'autre dans une direction axiale.

13. Connecteur à lumières multiples (1) selon l'une quelconque des revendications 1 à 12, où :
la première partie (2) comprend un premier connecteur supplémentaire disposé à l'intérieur du premier connecteur intérieur (2b) ;
la deuxième partie (3) comprend un deuxième connecteur supplémentaire disposé à l'intérieur du deuxième connecteur intérieur (3b) afin de fournir une troisième lumière disposée coaxialement à l'intérieur de la lumière intérieure (4).

14. Connecteur Luer à lumières multiples (1) selon l'une quelconque des revendications précédentes, comprenant en outre une première ligne de fluide (12) reliée fluidiquement à la lumière extérieure (4) et une deuxième ligne de fluide (13) reliée fluidiquement à la lumière intérieure (5).

15. Système (17) pour chirurgie ophtalmique comprenant :
le connecteur à lumières multiples (1) selon l'une quelconque des revendications 1 à 14, où :
la première partie (2) est pourvue dans un logement d'une cassette chirurgicale (18) comprenant un premier port et un deuxième port; et
la deuxième partie est couplée à une première ligne de fluide (12) via la première ouverture (8) et à une deuxième ligne de fluide (13) via la deuxième ouverture (9) ; et
où la première et deuxième ligne de fluide (12, 13) sont couplées respectivement à une première pointe chirurgicale (19) et à une deuxième pointe chirurgicale (20), et où les lumières extérieure et intérieure (4, 5) de la première partie (2) sont reliées fluidiquement au premier et au deuxième port de la cassette chirurgicale (18).
